# EUROPEAN PATENT SPECIFICATION

(11) **EP 3 531 127 B1**
(45) Date of publication and mention of the grant of the patent: **18.12.2024**
(21) Application number: 17862968.9
(22) Date of filing: 23.10.2017
(51) Int. Cl.: G01N 33/50, G01N 33/15, A61K 49/00, A01K 67/027

(54) **METHOD AND DEVICE FOR SCREENING DRUG**
VERFAHREN UND VORRICHTUNG FÜR WIRKSTOFF-SCREENING
PROCÉDÉ ET DISPOSITIF DE CRIBLAGE DE MÉDICAMENT

(30) Priority: 21.10.2016 CN 201610918458
(43) Date of publication of application: 28.08.2019
(73) Proprietor: Shanghai Lide Biotech Co., Ltd., Shanghai 201203 (CN)
(72) Inventor: WEN, Danyi, Shanghai 201203 (CN); ZHANG, Feifei, Shanghai 201203 (CN)
(74) Representative: Fabry, Bernd
(86) International application number: PCT/CN2017/107386
(87) International publication number: WO 2018/072758

(56) References cited:
- EP-A1- 0 218 400
- US-A- 5 676 924
- US-A- 5 698 413
- US-A1- 2002 090 690
- US-A1- 2004 067 540
- US-A1- 2007 172 425
- No further relevant documents disclosed
- ELIESER GORELIK ET AL.: "Microencapsulated Tumor assay: New Short-term Assay for in Vivo Evaluation of the Effects of Anticancer Drugs on Human Tumor Cell Lines", CANCER RESEARCH, vol. 47, 1 November 1987 (1987-11-01), pages 5739 - 5747, XP055477996
- QIU, YEFENG ET AL.: "The Application of Patient-derived Tumorxeno Graft Mouse Models Inprecision Medicine Inoncology", LABORATORY ANIMAL SCIENCE, vol. 33, no. 4, 31 August 2016 (2016-08-31), pages 78 - 83, XP009514102

## Description

### Technical field

The present invention relates to the field of *in vitro* drug screening, in particular to a method for determining the sensitivity of a primary tumor cell to an anti-tumor drug in a time-efficient manner and use of a kit in the method.

### Background

Individualized precision medicine has been widely used in clinical practice, especially for cancer treatment. Chemotherapy is a major treatment for patients with advanced metastatic cancer, but chemotherapeutic drugs lack specificity to tumor cells -- while killing tumor cells, they also kill a large number of bone marrow cells and other proliferating normal cells, causing serious adverse effects. At the same time, drug resistance is often produced after repeated chemotherapies, which will affect the effect of the chemotherapy. Therefore, the need for individualized precision medical treatment is becoming more and more urgent. Individualized precision medical treatment not only improves the accuracy of the treatment for a patient, but also effectively reduces the randomness and blindness of the treatment, so as to reduce the injury to the patient caused by drugs. In recent years, a patient-derived xenograft (PDX) model is widely used in precision medicine. There are many large clinical medical centers in North America using PDX models for pre-clinical drug development and drug screening, as well as for guiding individualized treatment for cancer patients.

The PDX model well maintains the biological characteristics the of primary tumor cells of a patient. The model is obtained by transplanting a fresh tumor tissue of a patient into an immune deficient mouse (e.g. a nude mouse or a severe combined immune deficiency (SCID) mouse). Such models maintain the genetic characteristics of the patient as well as tumor heterogeneity. Presently, the major difficulty of using PDX model for individualized precision medical treatment is the lengthy time period spent on modelling and sensitivity test, and the low success rate for modelling. For example, primary cell transplantation takes 2-3 months, and the drug sensitivity test takes 3-4 months. In addition, PDX modelling requires a high level of technical skills. Surgeons, histologists and researchers need to cooperate closely after the tumor sample is obtained from the patient. Therefore, how to improve the method for determining the sensitivity of tumor cells to an anti-tumor drug by using a PDX model becomes an urgent issue.
US5698413 (A) discloses a method of evaluating a chemotherapeutic agent using cells grown in a biocompatible, semi-permeable device. Specifically, cells are instilled into the encapsulation device, which is implanted directly into a laboratory animal. The animal is then treated with the test chemotherapeutic agent on a dose and schedule appropriate for the agent being evaluated. The implanted samples are allowed to remain in the host animal for an appropriate period of time. The implanted samples are then harvested for evaluation of the effects of the chemotherapeutic agent.
US 2004/067540 A1 discloses a rapid method for screening potentially pharmaceutically useful compounds for activity in vivo. The method has the steps of growing a target cell into which a reporter gene was introduced in a biocompatible, semipermeable encapsulation device; implanting the semi-permeable encapsulation device into a subject; administering a potentially pharmaceutically active compound to said subject; removing said encapsulation device from said subject after in vivo exposure to the potentially pharmaceutically active compound and evaluating said target cell for reaction to said potentially paharmaceutically active compound by measuring the expression of said reporter gene.
US 5676924A reports a method of determining the effectiveness of a cancer treatment by sealing tumor cells in segments of semipermeable membrane hollow fibers, implanting the sealed fiber segments in a mammal, treating the mammal with a cancer treatment, and evaluating the effect of the cancer treatment on the cells in the hollow fiber segments.

### Summary of the invention

To overcome the above mentioned technical problems, the present inventor has developed a method for determining the sensitivity of a tumor cell to an anti-tumor drug in a time-efficient and accurate manner.

One aspect of the present invention provides a method for determining the sensitivity of a primary tumor cell to an anti-tumor drug, including the following steps:
(1) isolating a tumor cell from an ex vivo tissue sample by digestion by 1X collagenase;
(2) sorting said tumor cell using CD45 cells sorting magnetic beads and fibroblasts sorting magnetic beads;
(3) transferring a primary tumor cell into an implant device;
(4) implanting the implant device comprising the primary tumor cell into a non-human animal;
(5) administering a candidate drug to the animal; and
(6) determining the sensitivity of the tumor cell to the candidate drug;
wherein the non-human animal is a mouse.

In embodiments, the primary tumor cell is obtained from an ex vivo tumor tissue sample. The ex vivo tumor sample can be obtained from a patient, or a PDX model established by using a tumor cell of the patient.

In embodiments, the mouse is a nude mouse.

In embodiments, the sensitivity of the tumor cell to the candidate drug is determined in vitro.

In embodiments, the candidate drug is administered to an animal orally or parenterally.

In embodiments, the sensitivity of the tumor cell to the candidate drug is determined 5-14 days after administering the candidate drug to the animal, preferably 5-7 days after administering the candidate drug to the animal.

In embodiments, the implant device is a tubular device having a molecular weight cut-off value of about 500,000 Dalton, preferably a modified polyvinylidene fluoride (PVDF) tube having a molecular weight cut-off value of about 500,000 Dalton, more preferably a modified PVDF tube with an inner diameter of about 1-2mm having a molecular weight cut-off value of about 500,000 Dalton.

One aspect of the present invention is to provide the use of a kit for determining the sensitivity of a primary tumor cell to an anti-tumor drug, wherein the kit comprises an implant device, said implant device is a tubular device with a molecular weight cut-off value of about 500,000 Dalton, preferably a modified polyvinylidene fluoride (PVDF) tube having a molecular weight cut-off value of about 500,000 Dalton, more preferably a modified PVDF tube with an inner diameter of about 1-2mm having a molecular weight cut-off value of about 500,000 Dalton.

### Figures

Figure 1 is a schematic diagram of the method of the present invention.
Figure 2 is a growth curve of primary gastric cancer cells obtained from a patient after administration of anti-tumor drug Tegafur in a traditional mouse PDX model GAPF155.
Figure 3 shows the pharmacodynamic data according to the method of the present invention of anti-tumor drug Tegafur (S-1) for gastric cancer cells from an *ex vivo* tumor tissue obtained from mouse PDX model GAPF155.
Figure 4 is a growth curve of primary gastric cancer cells obtained from patients after administration of anti-tumor drug Tegafur in mouse PDX model GAPF157.
Figure 5 shows the pharmacodynamic data according to the method of the present invention of anti-tumor drug Tegafur (S-1) for gastric cancer cells from an *ex vivo* tumor tissue obtained from mouse PDX model GAPF157.
Figure 6 is a growth curve of primary gastric cancer cells obtained from patients after administration of anti-tumor drug Tegafur in mouse PDX model GAPF161.
Figure 7 shows the pharmacodynamic data according to the method of the present invention of anti-tumor drug Tegafur (S-1) for gastric cancer cells from an *ex vivo* tumor tissue obtained from mouse PDX model GAPF161.
Figure 8 shows the results of a sensitivity test for primary lung adenocarcinoma cells from a patient to drugs according to the method and the kit of the present invention.
Figure 9 shows the results of a sensitivity test for primary duodenal cancer cells from a patient to drugs according to the method and the kit of the present invention.

### Detailed description of the invention

The present invention provides a method for determining the sensitivity of a primary tumor cell to an anti-tumor drug, comprising the following steps:
(1) isolating a tumor cell from an ex vivo tissue sample by digestion by IX collagenase;
2) sorting said tumor cell using CD45 cells sorting magnetic beads and fibroblasts sorting magnetic beads;
(3) transferring the primary tumor cell into an implant device;
(4) implanting the implant device comprising the primary tumor cell into a non-human animal;
(5) administering a candidate drug to the animal; and
(6) determining the sensitivity of the primary tumor cell to the candidate drug;
wherein the non-human animal is a mouse.

A primary tumor cell refers to a tumor cell obtained from an ex vivo tumor tissue sample. In embodiments, the primary tumor cell is isolated from a tumor tissue sample of a patient, including but not limited to a tumor tissue that is clinically removed, a tumor biopsy sample etc.. In embodiments, the primary tumor cell is obtained from a PDX mouse model which comprises a tumor cell from a tumor tissue of a patient.

The tumor cell can derive from tissue of various types of tumors, including but not limited to tumors located in the following parts of the body: the digestive tract (such as the stomach, intestine, duodenum, colon, pancreas, bile duct, anal canal, etc.), mammary glands, lung, liver, endocrine glands (such as adrenal gland, parathyroid gland, pituitary, testis, ovary, thymus, thyroid gland etc.), urinary and reproductive system (such as kidney, bladder, ovary, testis, prostate, etc.), skeletal muscle system (such as bone, smooth muscle, striated muscle, etc.), nervous system (such as brain), skin, head and neck, blood system and so on. For example, the primary tumor cell is derived from a gastric cancer tissue, a duodenal cancer tissue or a lung cancer tissue. The tumor cells can be derived from any type of tumors located in said parts of the body.

In embodiments, the primary tumor cell of the invention is a tumor cell that has been digested and sorted. In embodiments, digestion is carried out as follows: remove the non-tumor tissue and necrotic tissue, cut the tumor samples into small cubes, rinse with HBSS, collect the pellets and use 1X collagenase at 37°C to digest for 1-2 hours. In embodiments, sorting was carried out as follows: dilute with serum medium (1:1) to terminate digestion and run through 70 µ m screen mesh; collect cell suspension and centrifuge the suspension at 1000rpm for 3 minutes to remove supernatant, and re-suspended in PBS containing 1% FBS; adjust cell density to 1 × 10⁸/ml; add CD45 cells sorting magnetic beads and fibroblasts sorting magnetic beads at a concentration of 20 µ 1/10⁷ cells, incubate for 30min at room temperature. Cells are rinsed with PBS containing 1% FBS and re-suspended with 2ml PBS containing 1% FBS. The magnetic beads are mounted on the magnet column, and washed with PBS containing 1% FBS; then the re-suspended cells are loaded onto the magnetic column. After the liquid drains off, the column is washed twice with PBS containing 1% FBS, and the outflow liquid is collected. The collected liquid is centrifuged at 1000rpm for 3 minutes to remove the supernatant, suspended the precipitate in the culture medium, counted the number of cells, and adjusted the cell density to 1-10× 10⁵/ml.

The method for PDX mouse modelling is known in the art. For example, "Melanoma patient-derived xenografts accurately model the disease and develop fast enough to guide treatment decisions.", Oncotarget, Vol. 5, No. 20, Berglind O. Einarsdottir et al., published on September 8, 2014, and "Personalizing Cancer Treatment in the Age of Global Genomic Analyses: PALB2 Gene for cancer treatment in the global genome analysis: the mutation in the pancreatic cancer and the destruction of the pancreatic cancer. "Reaction of agents", Molecular Cancer Therapies, published for the first time on December 6, 2010; DOI: 10.1158/1535-7163.MCT-10-0893, Maria C. Villarroel et al.

The candidate drug may be a known anti-tumor drug or a combination of known anti-tumor drugs, a new anti-tumor drug or combination of new anti-tumor drugs, or a new combination of known anti-tumor drugs. In the method of the invention, the drugs to be measured may be used in the form of solid, semisolid, or liquid, and may be administered at a desirable frequency as required.

In the method of the present invention, the candidate drug can be administered to the animal orally or parenterally (such as via intravenous, intramuscular, subcutaneous or intravenous infusion), topical administration, inhalation, and transdermal delivery such as skin patches, implants, suppositories, etc. A skilled person in the art will choose a suitable route of administration according to needs.

In embodiments of the present invention, the sensitivity of the tumor cell to the candidate drug can be determined 5-14 days after administering the drug to the animal, preferably, 5-7 days after administering the drug to the animal. In the present invention, the sensitivity of the tumor cell to the candidate drug can be determined in vitro, such as by means of the following methods including but not limited to ATP detection, MTT, Brdu labelling, Ki67 staining and so on. Disclosed is an implant device used in the method of the invention. In embodiments, the implant device is a tubular implant device with a molecular weight cut-off value of about 500,000 Dalton. In a preferred embodiment, the tubular implant is a modified polyvinylidene fluoride tube, with a molecular weight cut off value of about 500,000 Dalton, more preferably the modified polyvinylidene fluoride tube has an inner diameter of about 1-2 mm. In embodiments of the invention, the implantation device can be implanted subcutaneously into the animals. A skilled person in the art will understand that a desired implantation method known in this field can be selected according to the need thereof.

Another aspect of the present invention provides the use of a kit, wherein said kit contains a modified polyvinylidene fluoride tube with an inner diameter of 1-2mm, which cuts off molecules with a molecular weight of around 500,000 Dalton. In embodiments, the kit also includes an insertion page, which includes the user manual for the kit.

The method of the present invention enables a primary tumor cells, especially an isolated and / or sorted tumor cells, to survive in the implant devices implanted in the experimental animals (such as mice), where the animal is a nutrition provider to enable the primary tumor cell to grow in the in vivo environment of the animal. The sensitivity of the tumor cell to anti-tumor drugs can be obtained rapidly and efficiently in vitro by detecting the growth state, the state of apoptosis and the degree of differentiation of the tumor cell after administering the drug to the animal. The results show that this method is highly correlated with the sensitivity results obtained by traditional PDX method. The invention can be combined with *in vivo* and *in vitro* experimental techniques to carry out rapid and efficient antitumor drug evaluation, greatly shortens the time needed for the traditional PDX model and saves the cost of clinical trials. In conclusion, the invention has the advantages of time-efficiency, convenient operation, low cost, good repeatability and applicability, in particular it realizes the rapid and accurate detection of the sensitivity of a tumor cell to antitumor drugs.

### Examples

For better illustration of the invention, the following is illustrated with reference to the accompanying drawings.

### Animals

4 to 5-week old female Nu/Nu mice was ordered from the supplier (purchased from Beijing Vital River Laboratory Animal Technology Co., Ltd.) and raised in SPF grade animal room. Before the experiment, the animal was adapted for at least three days.

### Sample collection

Freshly collected tumor samples obtained from surgery or from mouse PDX models containing tumor cells from patient were kept in a storage tube and transported to the laboratory on ice in the shortest possible time.

### Digestion and sorting

After removing the non-tumor tissues and necrotic tissues in the biosafety cabinet, the tumor samples were cut into cubes of 1-3 mm², rinsed with HBSS. Tumor tissue pellets were collected and centrifuged at 1000rpm for 3 minutes to remove the supernatant, and digested with 1X collagenase (purchased from Gibco) at 37 °C for 1-2 hours.

Diluted the digest mixture with serum medium (1:1) to terminate digestion and ran through 70 µ m screen mesh.

Collected cell suspension and centrifuged the suspension at 1000rpm for 3 minutes to remove supernatant, and re-suspended in PBS containing 1% FBS; counted cell numbers and adjusted cell density to 1 × 10⁸/ml.

Added CD45 cells sorting magnetic beads and fibroblasts sorting magnetic beads at 20 µ l/10⁷ cells, incubated for 30min at room temperature.

Cells were rinsed with PBS containing 1% FBS and re-suspended with 2ml PBS containing 1% FBS. The magnetic beads were mounted on the magnet column, and washed with PBS containing 1% FBS.

The re-suspended cells were loaded onto the magnetic column. After the liquid drained off, the column was washed twice with PBS containing 1% FBS, and the outflow liquid was collected.

The collected liquid was centrifuged at 1000rpm for 3 minutes to remove the supernatant and suspended in culture medium. Counted the number of cells, and adjusted the cell density to 1-10×10⁵/ml.

### Cell tubing, inoculation and viability test

Used a modified polyvinylidene fluoride tube with an inner diameter of 1mm as the implant device. The modified polyvinylidene fluoride tube has been pretreated by immersion, rinsing and high pressure sterilization. Intercepted the tube into segments of about 2 cm length, used PBS to repeatedly rinse 3-5 times, and blew off the liquid.

The suspension of tumor cells was added to the modified polyvinylidene fluoride tube and sealed.

The modified polyvinylidene fluoride (PVDF) tube was inoculated subcutaneously into the back of mice, and set the experiment groups and the control groups. The wound was glued with medical glue, and the mice were treated respectively.

5-14 day after administration, the mice were killed and cell viability was detected by quantitative analysis of ATP by CellTiter-Glo luminescence in the modified polyvinylidene fluoride tubes.

### Data analysis

According to the viability of tumor cells in the control group, the percentages of cell viable proliferation (t/c%) in the experiment groups were calculated, and then the efficacy of the corresponding drugs was evaluated.

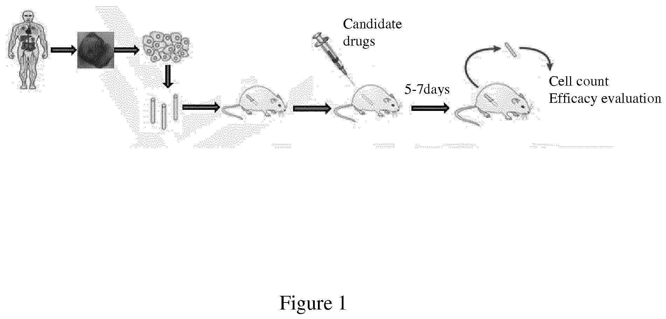

Figure 1 is a flow chart of the method of the invention for rapidly determining the efficacy of an anti-tumor drug. First, an *ex vivo* tumor tissues were obtained and then digested and sorted to obtain the isolated tumor cells, and the tumor cells were transferred into the implant device. The implanted devices were subcutaneously inoculated into the animals (such as mice), and removed 5-7 days after an anti-tumor drug was administered to the animal. The viability of the tumor cells was measured.

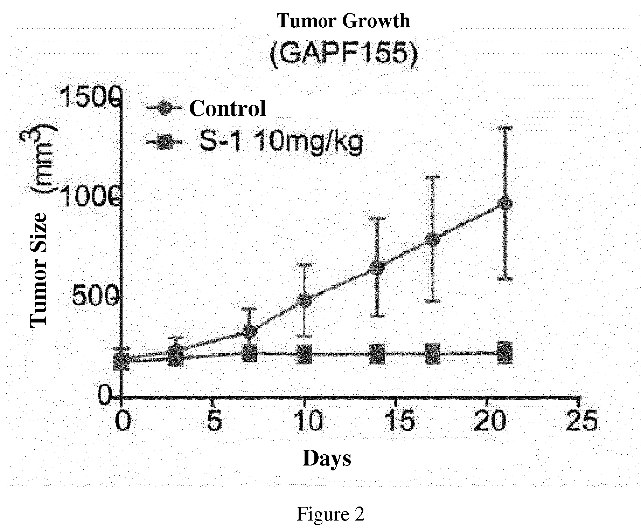

Figures 2-7 show a comparison of the results using a traditional PDX method and using the method of the invention.

### Example 1

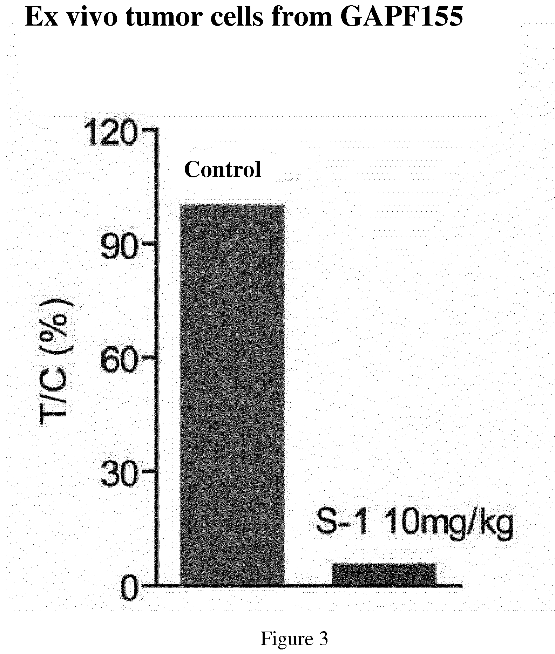

Mouse PDX model GAPF155 was obtained by implanting primary gastric cancer cells into mice. After 2 months of feeding, patient-derived gastric cancer cells were obtained from GAPF155. The sensitivity of the patient-derived gastric cancer cells in GAPF155 to S-1 was detected by the traditional PDX method and the method of the present invention (Fig. 2 and Fig. 3). The results showed that the tumor cells were sensitive to S-1.

### Example 2

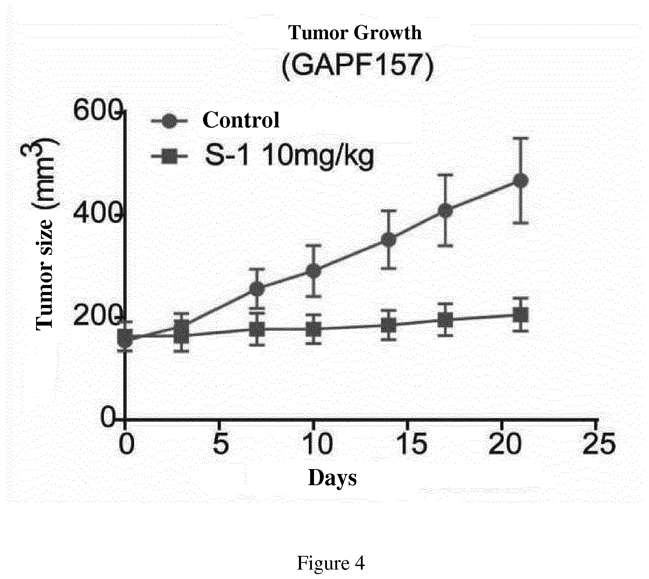

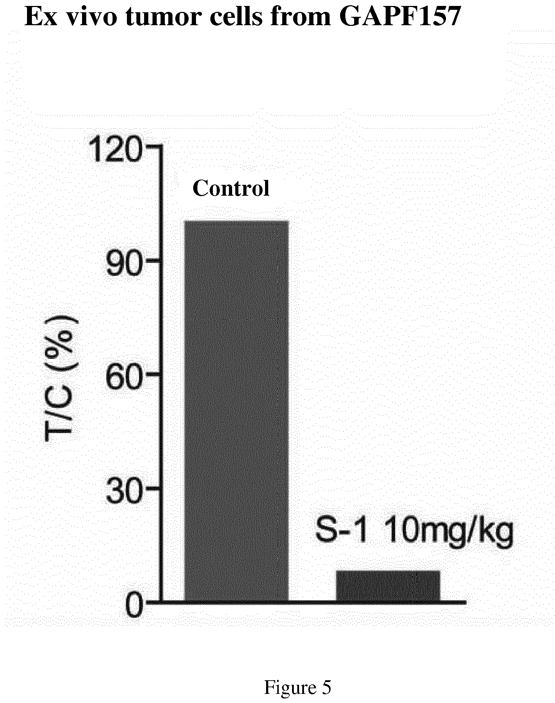

Mouse PDX model GAPF157 was obtained by implanting primary gastric cancer cells into mice. After 2 months of feeding, patient-derived gastric cancer cells were obtained from GAPF157. The sensitivity of the patient-derived gastric cancer cells in GAPF155 to S-1 was detected by the traditional PDX method and the method of the present invention (Fig.4 and Fig.5). The results showed that the tumor cells were sensitive to S-1.

### Example 3

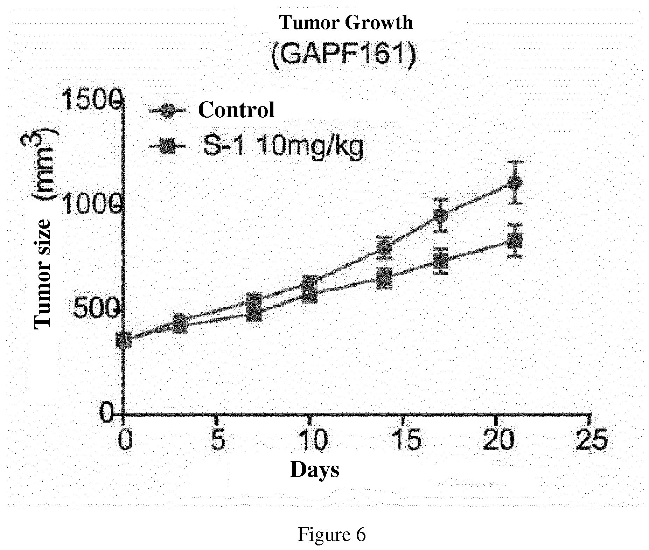

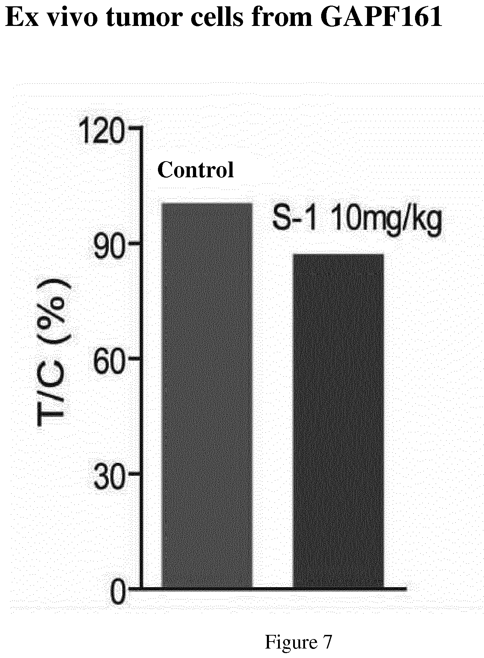

Mouse PDX model GAPF161 was obtained by implanting primary gastric cancer cells into mice. After 2 months of feeding, patient-derived gastric cancer cells were obtained from GAPF161. The sensitivity of the patient-derived gastric cancer cells in GAPF155 to S-1 was detected by the traditional PDX method and the method of the present invention (Fig.6 and Fig.7). The results showed that the tumor cells were sensitive to S-1.

The pharmacodynamic data obtained by the traditional PDX method and the method of the present invention showed that S-1 had good anti-tumor activity against the tumor cells in the GAPF155 model (FIGS. 2 and 3) and the tumor cells in the GAPF157 model (FIGS. 4 and 5), and S-1 had no obvious anti-tumor activity against the tumor cells in the GAPF161 model (FIGS. 6 and 7). The result obtained by the traditional PDX method was consistent with the result obtained by the present method.

### Example 4

Male patients with lung adenocarcinoma (60 years old) were selected as the candidate. According to the clinical experience for lung adenocarcinoma, the doctor chose Pemetrexed plus cisplatin. After 3 courses of treatment (9 weeks), the patient's condition recurred and the tumor started to metastate. With the patient's informed consent, primary tumor cells were obtained from pleural effusion of the patient.

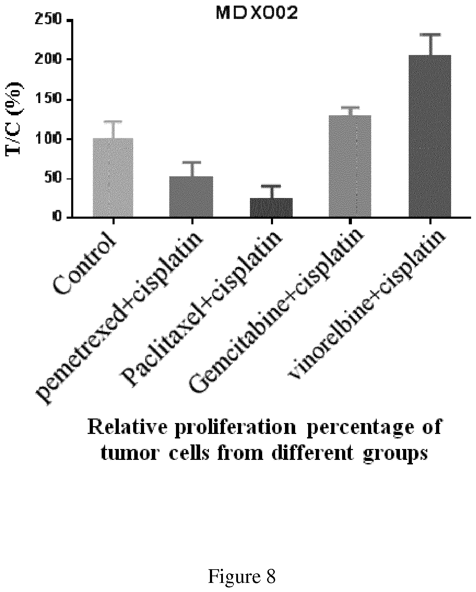

The sensitivity of the primary lung adenocarcinoma cell to the anti-tumor drugs was detected by the method of the present invention. The results showed that the regimen of paclitaxel plus cisplatin exhibited strong anti-tumor activity against tumor samples in this patient (Fig. 8).

### Results after adjusting the treatment:

The therapeutic regimen was changed to paclitaxel plus cisplatin by doctor. After 7 weeks of treatment, the pleural effusion disappeared and the patient was in stable condition and dismissed from hospital.

### Example 5

A male patient with duodenal ampullary tumor was selected (56 years old). After the patient's informed consent, the primary tumor tissue was obtained from the patient.

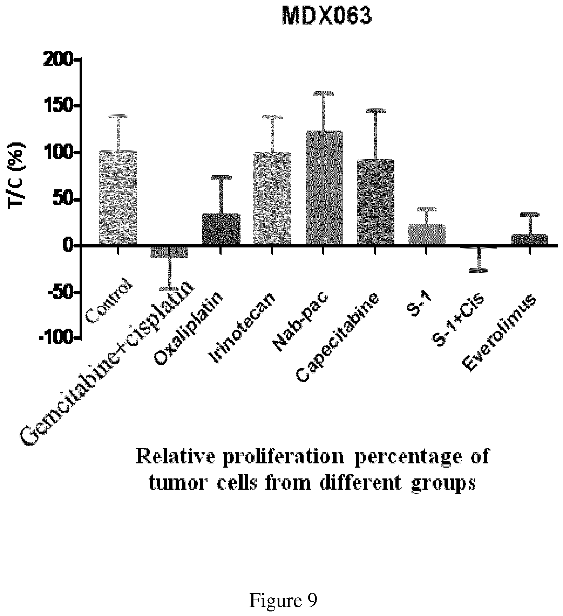

Drug screening was carried out according to the method of the present invention. The results showed that patient well responded to gemcitabine for treatment of pancreatic cancer (Fig. 9).

The doctor selected gemcitabine plus cisplatin based on the above results, and the patient's condition was under control.

The following table 1 summarizes the results of the method of the present invention for determining the sensitivity of various types of primary cancer cells to anti-tumor drugs in patients.

**Table 1**

| **Type of cancer cells** | **Number of cases** | | **Type of cancer cells** | **Number of cases** | |
|---|---|---|---|---|---|
| Lung cancer | | 72 | Gastric cancer | | 14 |
| Cholangiocarcinoma | | 28 | Pancreas cancer | | 35 |
| Gallbladder carcinoma | | 19 | Liver cancer | | 15 |
| Osteosarcoma | | 1 | Head and neck cancer | | 5 |
| Colon cancer | | 23 | Ovary cancer | | 3 |
| Glioma | | 10 | Bladder cancer | | 2 |
| Carcinoma of duodenum | | 3 | Trophoblastoma | | 4 |
| Esophageal cancer | | 8 | Adenoid cystic carcinoma | | 1 |
| Endometrial carcinoma | | 3 | Testicular cancer | | 1 |
| Breast cancer | | 4 | Prostate cancer | | 4 |
| Anal canal cancer | | 1 | Rectal cancer | | 5 |
| total | | | 261 (cases) | | |

The above examples are only described by way of illustration. Without deviating from the scope of protection specified in the appended claims of the present invention, variants may apply.

## Claims

1. A method for determining the sensitivity of a primary tumor cell to an anti-tumor drug, comprising the following steps:
(1) isolating a tumor cell from an *ex vivo* tissue sample by digestion by 1X collagenase;
(2) sorting said tumor cell using CD45 cells sorting magnetic beads and fibroblasts sorting magnetic beads;
(3) transferring the primary tumor cell into an implant device;
(4) implanting the implant device comprising the primary tumor cell into a non-human animal;
(5) administering a candidate drug to the animal; and
(6) determining the sensitivity of the primary tumor cell to the candidate drug;
wherein the non-human animal is a mouse.

2. The method of claim 1, wherein the *ex vivo* tumor tissue sample is obtained from a patient.

3. The method of claim 1, wherein the ex vivo tumor tissue sample obtained from a patient-derived xenograft (PDX) model established using a tumor cell of a patient.

4. The method of any one of claims 1-3, wherein the mouse is a nude mouse.

5. The method of any one of claims 1-4, wherein the sensitivity of the tumor cell to the candidate drug is determined in vitro.

6. The method of any one of claims 1-5, wherein the candidate drug is administered to the animal orally or parenterally.

7. The method of any one of claims 1-6, wherein the sensitivity of the tumor cell to the candidate drug is determined 5-14 days after administering the candidate drug to the animal.

8. The method of any one of claims 1-6, wherein the sensitivity of the tumor cell to the candidate drug is determined 5-7 days after administering the candidate drug to the animal.

9. The method of any one of claims 1-8, wherein the implant device is a tubular device having a molecular weight cut-off value of about 500,000 Dalton.

10. The method of any one of claims 1-8, wherein the implant device is a modified polyvinylidene fluoride (PVDF) tube having a molecular weight cut-off value of about 500,000 Dalton.

11. The method of any one of claims 1-8, wherein the implant device is a modified PVDF tube with an inner diameter of about 1-2mm having a molecular weight cut-off value of about 500,000 Dalton.

12. The method of any one of claims 1-11, wherein the implant device is implanted into the animal subcutaneously.

13. Use of a kit for determining the sensitivity of a primary tumor cell to an anti-tumor drug by carrying out the method according to any one of claims 1-12, wherein the kit comprises an implant device, wherein the implant device is a tubular device having a molecular weight cut-off value of about 500,000 Dalton.

14. The use of claim 13, wherein the implant device is a modified polyvinylidene fluoride (PVDF) tube having a molecular weight cut-off value of about 500,000 Dalton.

15. The use of claim 13, wherein the implant device is a modified PVDF tube with an inner diameter of about 1-2mm having a molecular weight cut-off value of about 500,000 Dalton.

## Patentansprüche

1. Verfahren zur Bestimmung der Empfindlichkeit einer primären Tumorzelle gegenüber einem Anti-Tumor-Medikament, umfassend die folgenden Schritte:
(1) Isolieren einer Tumorzelle aus einer *Ex-vivo-Gewebeprobe* durch Verdauung mit 1X Kollagenase;
(2) Sortieren der Tumorzelle unter Verwendung von CD45-Zellen sortierenden Magnetperlen und Fibroblasten sortierenden Magnetperlen;
(3) Überführen der primären Tumorzelle in eine Implantationsvorrichtung;
(4) Implantieren der Implantationsvorrichtung, die die primäre Tumorzelle enthält, in ein nicht-menschliches Tier;
(5) Verabreichen eines Arzneimittelkandidaten an das Tier; und
(6) Bestimmen der Empfindlichkeit der primären Tumorzelle gegenüber dem Arzneimittelkandidaten;
wobei das nicht-menschliche Tier eine Maus ist.

2. Verfahren nach Anspruch 1, wobei die *Ex-vivo-Tumorgewebeprobe* von einem Patienten erhalten wird.

3. Verfahren nach Anspruch 1, wobei die Ex-vivo-Tumorgewebeprobe aus einem von einem Patienten stammenden Xenotransplantationsmodell (PDX) gewonnen wird, das unter Verwendung einer Tumorzelle eines Patienten hergestellt wurde.

4. Verfahren nach einem der Ansprüche 1-3, wobei die Maus eine Nacktmaus ist.

5. Verfahren nach einem der Ansprüche 1-4, wobei die Empfindlichkeit der Tumorzelle gegenüber dem Arzneimittelkandidaten in vitro bestimmt wird.

6. Verfahren nach einem der Ansprüche 1 bis 5, wobei der Wirkstoffkandidat dem Tier oral oder parenteral verabreicht wird.

7. Verfahren nach einem der Ansprüche 1-6, wobei die Empfindlichkeit der Tumorzelle gegenüber dem Arzneimittelkandidaten 5-14 Tage nach Verabreichung des Arzneimittelkandidaten an das Tier bestimmt wird.

8. Verfahren nach einem der Ansprüche 1-6, wobei die Empfindlichkeit der Tumorzelle gegenüber dem Arzneimittelkandidaten 5-7 Tage nach Verabreichung des Arzneimittelkandidaten an das Tier bestimmt wird.

9. Verfahren nach einem der Ansprüche 1-8, wobei die Implantatvorrichtung eine röhrenförmige Vorrichtung mit einem Molekulargewichts-Cut-off-Wert von etwa 500.000 Dalton ist.

10. Verfahren nach einem der Ansprüche 1 bis 8, wobei die Implantationsvorrichtung ein modifiziertes Polyvinylidenfluorid (PVDF)-Rohr mit einem Molekulargewichts-Cut-off-Wert von etwa 500.000 Dalton ist.

11. Verfahren nach einem der Ansprüche 1 bis 8, wobei die Implantationsvorrichtung ein modifizierter PVDF-Schlauch mit einem Innendurchmesser von etwa 1 bis 2 mm und einem Molekulargewichts-Cutoff-Wert von etwa 500.000 Dalton ist.

12. Verfahren nach einem der Ansprüche 1-11, wobei die Implantationsvorrichtung dem Tier subkutan implantiert wird.

13. Verwendung eines Kits zur Bestimmung der Empfindlichkeit einer primären Tumorzelle gegenüber einem Anti-Tumor-Medikament durch Durchführung des Verfahrens nach einem der Ansprüche 1-12, wobei das Kit eine Implantationsvorrichtung umfasst, wobei die Implantationsvorrichtung eine röhrenförmige Vorrichtung mit einem Molekulargewichts-Cut-off-Wert von etwa 500.000 Dalton ist.

14. Verwendung nach Anspruch 13, wobei die Implantationsvorrichtung ein modifiziertes Polyvinylidenfluorid (PVDF)-Rohr mit einem Molekulargewichts-Cut-off-Wert von etwa 500.000 Dalton ist.

15. Verwendung nach Anspruch 13, wobei die Implantationsvorrichtung ein modifizierter PVDF-Schlauch mit einem Innendurchmesser von etwa 1-2 mm ist, der einen Molekulargewichts-Cut-off-Wert von etwa 500.000 Dalton aufweist.

## Revendications

1. Une méthode pour déterminer la sensibilité d'une cellule tumorale primaire à un médicament antitumoral, comprenant les étapes suivantes :
(1) isolement d'une cellule tumorale à partir d'un échantillon de tissu *ex vivo* par digestion au moyen d'une collagénase 1X ;
(2) trier ladite cellule tumorale à l'aide de billes magnétiques de tri des cellules CD45 et de billes magnétiques de tri des fibroblastes ;
(3) transfert de la cellule tumorale primaire dans un dispositif d'implantation ;
(4) implanter le dispositif d'implantation comprenant la cellule tumorale primaire dans un animal non humain ;
(5) l'administration d'un médicament candidat à l'animal ; et
(6) déterminer la sensibilité de la cellule tumorale primaire au médicament candidat ;
où l'animal non humain étant une souris.

2. Méthode selon de la revendication 1, dans laquelle l'échantillon de tissu tumoral ex *vivo* est obtenu à partir d'un patient.

3. Méthode selon de la revendication 1, dans laquelle l'échantillon de tissu tumoral ex vivo provient d'un modèle de xénogreffe dérivée d'un patient (PDX) établi à l'aide d'une cellule tumorale d'un patient.

4. Méthode selon de l'une des revendications 1 à 3, dans laquelle la souris est une souris nue.

5. Méthode selon de l'une des revendications 1 à 4, dans laquelle la sensibilité de la cellule tumorale au médicament candidat est déterminée in vitro.

6. Méthode selon l'une des revendications 1 à 5, dans laquelle le médicament candidat est administré à l'animal par voie orale ou parentérale.

7. Méthode selon l'une quelconque des revendications 1 à 6, dans lequel la sensibilité de la cellule tumorale au médicament candidat est déterminée 5 à 7 jours après l'administration du médicament candidat à l'animal.

8. Méthode selon de l'une des revendications 1 à 6, dans laquelle la sensibilité de la cellule tumorale au médicament candidat est déterminée 5 à 7 jours après l'administration du médicament candidat à l'animal.

9. Méthode selon de l'une des revendications 1 à 8, dans laquelle le dispositif d'implantation est un dispositif tubulaire ayant une valeur de coupure de poids moléculaire d'environ 500 000 Dalton.

10. Méthode selon de l'une des revendications 1 à 8, dans laquelle le dispositif d'implantation est un tube en polyfluorure de vinylidène (PVDF) modifié dont la valeur de coupure du poids moléculaire est d'environ 500 000 Dalton.

11. Méthode selon de l'une des revendications 1 à 8, dans laquelle le dispositif d'implantation est un tube en PVDF modifié d'un diamètre intérieur d'environ 1 à 2 mm ayant une valeur de coupure de poids moléculaire d'environ 500 000 Dalton.

12. Méthode selon de l'une des revendications 1 à 11, dans laquelle le dispositif d'implantation est implanté dans l'animal par voie sous-cutanée.

13. Utilisation d'un kit pour déterminer la sensibilité d'une cellule tumorale primaire à un médicament antitumoral en mettant en oeuvre la méthode selon l'une des revendications 1 à 12, dans lequel le kit comprend un dispositif d'implantation, dans lequel le dispositif d'implantation est un dispositif tubulaire ayant une valeur de coupure de poids moléculaire d'environ 500 000 Dalton.

14. Utilisation selon de la revendication 13, dans laquelle le dispositif d'implantation est un tube en polyfluorure de vinylidène (PVDF) modifié ayant une valeur de coupure de poids moléculaire d'environ 500 000 Dalton.

15. Utilisation selon la revendication 13, dans laquelle le dispositif d'implantation est un tube PVDF modifié d'un diamètre intérieur d'environ 1 à 2 mm ayant une valeur de coupure de poids moléculaire d'environ 500 000 Dalton.
